# EUROPEAN PATENT APPLICATION

(11) **EP 2 510 932 A1**
(43) Date of publication of application: **17.10.2012**
(21) Application number: 11162632.1
(22) Date of filing: 15.04.2011
(51) Int. Cl.: A61K 35/74, A23L 1/30, A61P 37/08

(54) **Lactobacillus paracasei NCC2461 (ST11) for use by perinatal maternal administration in the reduction and prevention of allergies in progeny**

(71) Applicant: Nestec S.A., 1800 Vevey (CH)
(72) Inventor: Mercenier, Annick, 1030, BUSSIGNY (CH); Zuercher, Adrian, 3018, BERN (CH); Nutten, Sophie, 1005, LAUSANNE (CH); Wiedermann, Ursula, 1130, VIENNA (AT); Schabussova, Irma, 1090, VIENNA (AT)
(74) Representative: Corticchiato, Olivier

(57) **Abstract**

The present invention provides a probiotic, *Lactobacillus paracasei* NCC 2461, i.e. ST11, for use by administration to expectant females and/or lactating mothers, and to their progeny for the reduction or prevention of the development of allergic immune responses in progeny. A daily dose of ST11 is administered to a pregnant woman for at least two weeks before delivery and, after delivery, the daily dose of ST11, is administered to the newborn infant for at least two months, either directly or via the mother's milk. The infant immune responses to allergens are thus reduced, resulting in a "dampened" allergic response. Thus allergy, including atopy, may be prevented in the progeny.

## Description

### Field of the Invention

This invention relates to perinatal administration of probiotic bacteria capable of modulating the infant immune response to allergens.

### Background to the Invention

The prevalence of allergic diseases increased rapidly within the last decades. As by now over a third of the worldwide population is afflicted, allergy has been considered as the new epidemic of the industrialized countries. The reasons for the steady increase in allergic diseases are not yet fully understood. Genetic background of the host is a prominent factor, and recently discovered genes have been shown to be associated with respiratory allergies/asthma and skin symptoms [Holloway, J.W. et al. (2010); Genetics of Allergic Disease, J.AC.I., 125: S81-94]. Environmental factors such as lifestyle, pollution, decreasing family size, and reduction of microbial stimulation of the immune system in early life stage as a consequence of an improved hygienic situation seem to play an important role also.

Allergic sensitization in childhood, especially in early childhood and especially to food allergens, is critical and of highest interest as development of an "allergic phenotype" or "atopy" has been shown to facilitate subsequent adverse reactions to other allergens. Hence allergies in childhood can be the first step of an allergic cascade leading to multiple allergies later in life, a situation commonly referred to as "The Atopic March". For example, it has been demonstrated in human cohorts that children with persistent food hypersensitivity early in life have a dramatically increased risk to develop allergic rhinitis (hay fever) or asthma later in childhood (Östblom, E. et al. (2008); Phenotypes of food hypersensitivity and development of allergic diseases during the first 8 years of life, Clinical and Experimental Allergy, 38 (8): 1325-1332). Therefore, preventing onset or attenuating the severity of food hypersensitivity may be crucial for slowing down the "Atopic March". In this context the management of allergic episodes and moreover prevention of allergies are, in childhood and infancy, of the highest importance.

### Prevention of allergies can be achieved on different levels

"Primary prevention" is the effect of preventing or reducing the risk of sensitization of patients to allergens, characterized by absence or reduced levels of allergen-specific IgE antibodies. Preventing or reducing sensitization will result in absence or reduction of allergic symptoms upon re-exposure to the same allergen. The current invention is concerned with primary prevention of allergies.

"Secondary prevention" is the effect of modulating the symptoms of allergies, i.e. the occurrence or intensity of the allergic reaction in patient already sensitized to one or several allergens when the patient is re-exposed to said allergen(s). By modulating the occurrence or intensity of the allergic symptoms (secondary prevention), the negative impact on the quality of life of the allergy sufferer, that is associated with allergies, is minimized.

Given these distinct concepts of allergy prevention it may be hypothesized that by virtue of their inherent mechanisms of action, some compounds might act solely at one or at both of these specific levels of prevention. Some may, for example, solely reduce the risk of the sensitization to a specific allergen (primary prevention), while other compounds may solely have an effect on the secondary prevention and reduce the severity of allergic reactions. Other compounds may be able to influence both sensitization and symptoms and thus are effective in promoting primary and secondary prevention.

Food allergens are among the first allergens that infants encounter in their early life: typically, cow's milk proteins may be encountered by infants not receiving exclusive breast-feeding. Milk-proteins are indeed among the most frequently observed causes for food allergy in infancy, followed by eggs and wheat proteins. In general, food allergies can manifest by cutaneous (rash, eczema, others) and gastrointestinal symptoms (abdominal cramps; pain, especially in the abdomen; vomiting) in infants and young children. Further sensitization and episodes of allergies can also appear when the infant/young child is exposed to a novel food such as cereals, vegetables, fruits, nuts or fish and also to air borne allergens such as pollen.

Air borne allergens such as pollen, house dust mites and animal dander are also a major cause of allergy in children and adults. It is known that birch pollen-allergic people frequently present allergic symptoms after ingestion of several kinds of plant-derived foods [Vieths, S., Scheurer, S. and Ballmer-Weber, B. (2002); Current Understanding of Cross-Reactivity of Food Allergens and Pollen, Annals of the New York Academy of Sciences, 964; 47-68.]. The majority of these reactions is caused by four distinct cross-reactive protein structures that are present in birch pollen. Proteins that share common epitopes with Bet v 1, the major birch pollen allergen, occur in pollens of several tree species and in fruits and vegetables, such as: apples, stone fruits, celery, carrot, nuts, and soybeans. It was shown in the study of Vieths et al. that approximately 70% of patients studied who are allergic to birch pollen may experience symptoms after consumption of foods from these groups. Thus, development of allergies to air borne allergens like birch pollen is also directly linked to food allergy manifestations, known as cross reactivity in oral syndrome.

### Gastrointestinal Microbiota and the immune System

Commensal gastrointestinal microbes constitute the earliest and most substantial stimulus for the development of the gut associated lymphoid tissue and associated immune system.

There is solid evidence from epidemiological studies that Western-type living conditions, e.g. reduced consumption of fermented food, substantial use of antibiotics and other drugs, and increased hygiene, are associated with the rise in allergic diseases. The so-called "hygiene hypothesis" thus suggests that a lack of exposure to microbial stimulus early in childhood is a major factor involved in this trend [Von Mutius, E. (2007); Allergies, Infections and the hygiene hypothesis - The epidemiological evidence, Immunobiology, 212(6); 433-9].

Indeed, epidemiological studies have demonstrated an association between the development of allergic diseases and disturbance of the gastrointestinal microbiota. For example, atopic children in Western societies have been reported to be less frequently colonized with lactobacilli or bifidobacteria than healthy children [Suzuki, S., Shimojo, N., Tajiri, Y., Kumemura, M., Kohno, Y. (2007); Differences in the composition of intestinal Bifidobacterium species and the development of allergic diseases in infants in rural Japan, Clin Exp Allergy; 37; 506-511; Ouwehand, A.C., Isolauri, E., He, F., Hashimoto, H., Benno, Y., Salminen, S. (2001); Differences in Bifidobacterium flora composition in allergic and healthy infants, J. Allergy Clin. Immunol. 108; 144-145; Kalliomaki, M., Salminen, S., Arivilommi, H., Kero, P., Koskinen, P., and Isolauri, E. (2001); Probiotics in primary prevention of atopic disease: a randomised placebo- controlled trial, Lancet 357: 1076-9].

Interestingly, clearcut differences exist with respect to the composition of the gut microbiota in response to the infant's feeding. The faecal microbiota of breast-fed infants includes appreciable populations of bifidobacteria with some *Lactobacillus* species, whereas formula-fed infants have more complex microbiota, with *Bifidobacterium* species and *Bacteroides* species, clostridia and streptococci being usually present. After weaning, a pattern of gut microbiota resembling that of an adult pattern becomes established over time [Penders, J., et al. (2006); Gut microbiota composition and development of atopic manifestations in infancy: the KOALA birth cohort study. Gut 56: 661-667].

These findings provide a rational for therapies to prevent allergic disease by supplementation of human diet with probiotic bacteria. Furthermore, as it is known that breast fed children have a lower frequency and/or severity of allergies than do formula fed children, thus supporting probiotic interventions that promote a gut microbiota in non-breast fed children that resembles as much as possible that of breastfed children.

There is also evidence that immunoprogramming by environmental factors may act within a narrow window of opportunity, either during pregnancy or early childhood when immune responses are still developing [Aaltonen, J., Ojala, T., Laitinen, K., Poussa, T., Ozanne, S., Isolauri, E. (2011); Impact of maternal diet during pregnancy and breastfeeding on infant metabolic programming: a prospective randomized controlled study. E. Eur. J. Clin. Nutr. 65; 10-19].

Thus, the immunological status of the newborn is important. This status includes the protection present at the birth of the infant and the acquisition of such immunological protection during the first hours, days or weeks of the infant life. The ability to acquire and maintain such protection is a crucial factor in the health of the infant faced with his new environment. Thus, pre-, peri-, and/or postnatal interventions correspond to a promising approach to modulate immune responses promoting and/or sustaining a non-atopic status. Moreover, interventions during pregnancy/lactation may have considerable advantages in terms of convenience and compliance compared to child-directed interventions.

However, recent reports confirm that peri- and/or post-natal probiotic treatment does not yield predictable results. For example, in WO 01/97822 it is disclosed that, *Lactobacillus rhamnosus* GG, given to mothers starting 2-4 weeks before delivery and during the breastfeeding period or directly to infants in the first 6 months of life reduced the incidence of eczema at 2 years by around 50% in children who were at high risk. However, contradictory results for the same strain were reported in a paper from Kopp et *al*. in 2008 [Kopp et al., (2008); Randomized, Double Blind, Placebo Controlled Trial of probiotics for Primary Prevention : No clinical Effects of Lactobacillus GG supplementation Pediatrics, vol. 121 no. 4].

*Lactobacillus rhamnosus* GG administration to mothers 4 to 6 weeks before expected delivery and then to the offspring during a period of 6 months neither reduced the incidence of atopic dermatitis nor altered the severity of atopic dermatitis in affected children. The paper concluded that *Lactobacillus rhamnosus* GG cannot be generally recommended for primary prevention. These experiments were carried out on a specific sub-group of the general population, i.e. progeny at high risk of allergy.

In WO2009/118243 it was disclosed that specific probiotic microorganisms administered to female mice during the gestation period or the lactation period or directly administered to progeny after birth boosted the immunity of the progeny. Thus, administration of *Lactobacillus rhamnosus* CGMCC 1.3724 (LPR) increased peripheral IgG responses to measles vaccine in the mice pups. However, the effect was specific to the strain used.

Thus, in the light of these cited prior art documents, there is a need for a further progress in area of probiotic administration as a way to improve the health of newborn babies and young infants.

There is therefore a need to positively impact the health of the progeny by a targeted nutritional diet of the expecting mothers.

There is, in particular, a need to help guaranteeing the best immune system in the progeny, in order to best prepare them to the early life antigenic challenges as well as to enhance the future maturation of their immune system to better promote protection during later infancy.

There is a need to impact the building of the immune system of progeny at the earliest possible stage during gestation as well as during the early phases of their newborn life when the immune system is rapidly maturing.

### Summary of the invention

The present invention provides a probiotic, *Lactobacillus paracasei* NCC 2461, i.e. ST11, for use by administration to expectant females and/or lactating mothers, and to their progeny for the reduction or prevention of the development of allergic immune responses in progeny. The reduction in allergic immune response is with respect to progeny of mothers not treated with ST11.

The progeny may be those at risk of atopic diseases. The allergic immune responses concerned by the invention are to food or airborne allergens, especially birch pollen allergens Bet v 1 and Bet v 2. The allergic immune responses may be allergen-specific recall responses or non allergen -specific (mitogen)-induced responses. The reduction in the allergy immune response may be manifested as a reduction in IL-4 and IL-5 production, in the case of allergen specific recall responses, and/or a reduction in IL-5 production by committed lymphocytes in the case of mitogen-induced responses.

The infant immune responses to allergens are thus reduced, resulting in a "dampened" allergic response in the progeny. Thus allergy, including atopy, may be prevented in the progeny.

According to the invention, the ST11 is administered to expectant mothers for at least two weeks before delivery and, after delivery, to the progeny either directly or via mothers milk, for at least 2 months, preferably up to six months or even up to one year after delivery.

Thus, the mother receives the ST11 as a daily dose during at least part or all of her pregnancy and, after delivery, during at least part or all of the lactation period if she is lactating. The ST11 may be administered orally to the expectant or lactating mothers, preferably in foods, drinks, dietary supplements or pharmaceutical compositions.

A daily dose of ST11 is administered to the newborn infant for at least two months, preferably up to six months, after delivery either directly or via the mother's milk. It may be administered in its pure form or diluted in water, breast milk or in an infant formula.

The daily dose of ST11 that is administered to the expectant or lactating mother is from 1x10⁶ to 1x10¹¹ cfu, preferably 1x10⁸ to 1x10¹⁰ cfu (cfu = colony forming unit), whereas the daily doses administered the newborn infant are from 1x10² to1x10¹⁰, preferably 1x10² to1x10⁴ cfu (cfu = colony forming unit).

The invention also concerns a method for primary prevention or reduction of the development of allergic immune responses in infants, comprising
- administering to a pregnant woman a daily dose of live *Lactobacillus paracasei* NCC 2461 (ST11) for at least two weeks before delivery, and
- after delivery, administering to the newborn infant a daily dose of live probiotic bacteria for at least 2 months, preferably up to six months or one year after delivery.

### Brief Description of the Drawings

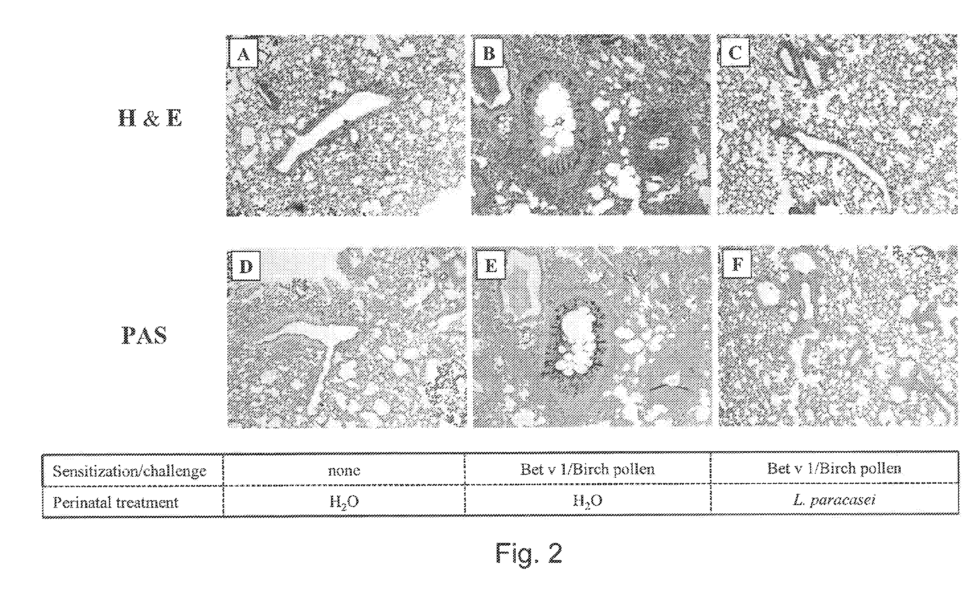

**Figure 1****:** Experimental Design
   Scheme of the experimental mouse model (Female BALB/c mice; n = 8) of birch pollen allergy used to demonstrate that maternal supplementation with *L. paracasei* ST11 protects progeny from the development of an allergic phenotype.
**Figure 2****:** Histology
   Paraffin-embedded sections of whole lungs fixed stained with hematoxylin and eosin (H&E) stain (Fig. **2A, B** and **C**) and with periodic acid-Schiff-stained (PAS) (Fig. **2D, E** and **F**). Panels **A** and **D** correspond to non sensitized/non challenged and non probiotic-treated mice; panels **B** and **E** correspond to mice sensitized with Bet v 1 and challenged with birch pollen; panels **C** and **E** correspond to mice sensitized with Bet v 1 and challenged with birch pollen and having received a treatment with *L. paracasei* ST11 perinatally.

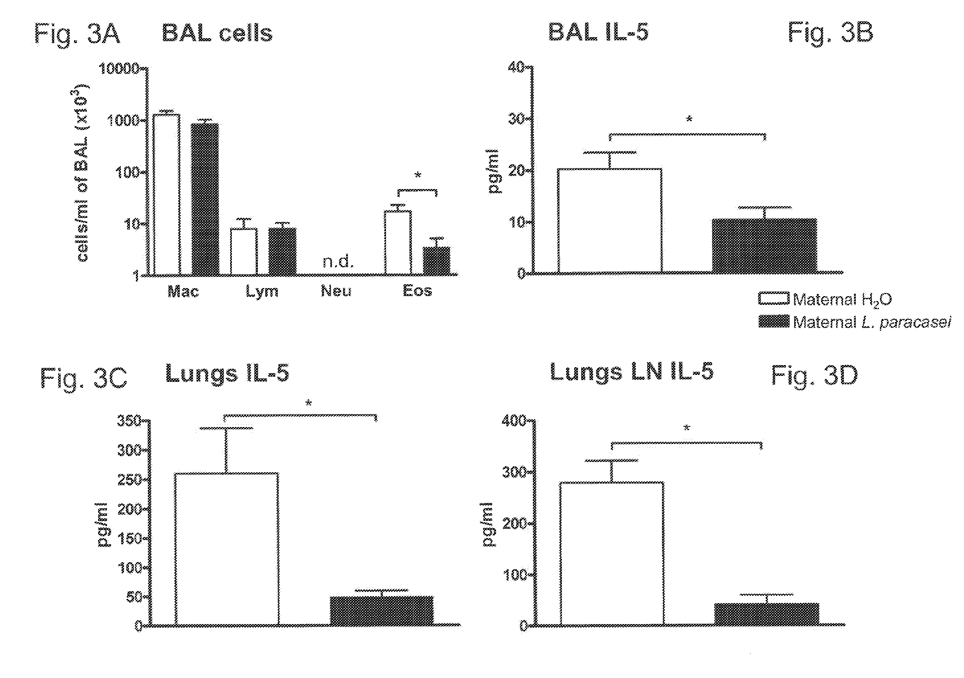

**Figure 3****:** Lung inflammation in progeny mice
   The effect of ST11 perinatal treatment on lung inflammation. Panel **A** shows cellular infiltration (macrophages, lymphocytes, neutrophils and eosinophils counts) in bronchoalveolar lavage (BAL) fluid of progeny. Panels **B, C** and **D** show levels of interleukin 5 (IL-5) in BAL fluid, lung and lung draining mediastinal lymph nodes, respectively. White bars correspond to progeny (sensitized and challenged to birch pollen) of mothers which were not treated with *L. paracasei* ST11. Black bars correspond to progeny (sensitized and challenged to birch pollen) of mothers which were treated with ST11.

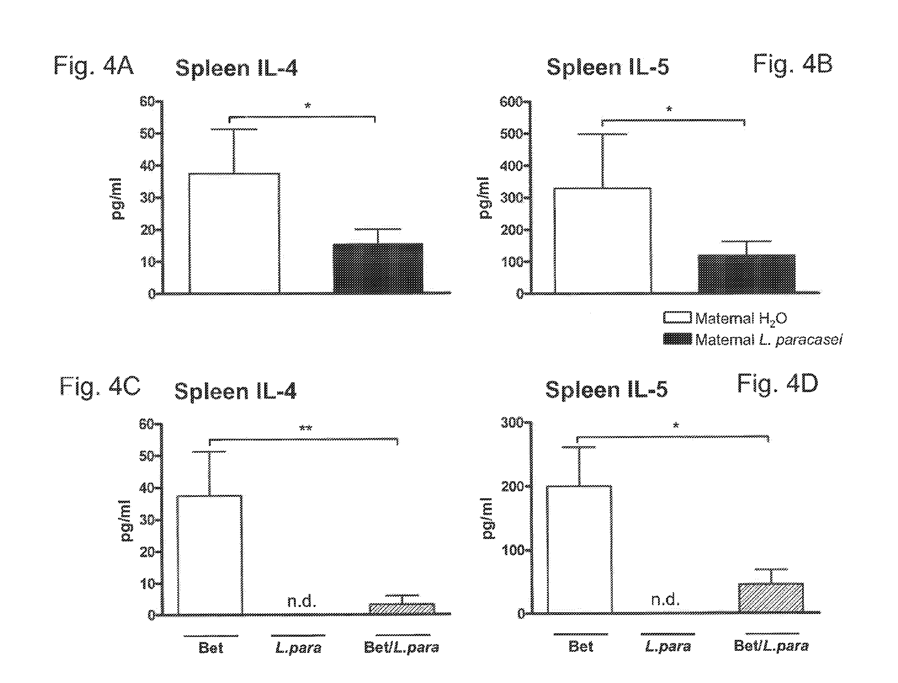

**Figure 4****:** Cytokine production after ex *vivo* stimulation of splenocytes
   Levels of Th2 cytokines IL-4 **(A)** and IL-5 **(B)** secretion by *ex vivo* allergen-restimulated spleen cell cultures from the sensitized and challenged progeny of *L. paracasei* ST11-supplemented mothers (black bars) compared with sensitized and challenged progeny derived from control mothers (white bars). Levels of Th2 cytokines IL-4 **(C)** and IL-5 **(D)** secretion by spleen cells cultures ex *vivo* restimulated with rBet v 1 (10µg/ml). Immunomodulatory potential of *L. paracasei* ST11 was assessed using spleen cells of offspring of sham (control)-treated mothers: splenocytes were incubated with *L. paracasei* ST11 (10⁸ cfu/ml) admixed to rBet v 1 (10µg/ml) for 48 h.

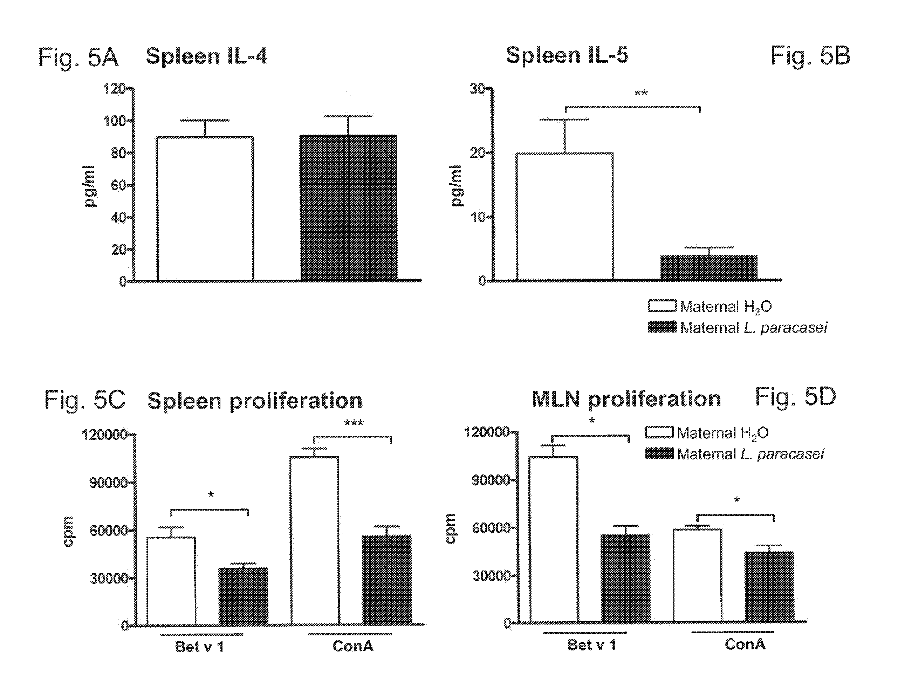

**Figure 5****:** Cytokine production after ex *vivo* stimulation of splenocytes
   Levels of IL-4 (**A**) and IL-5 (**B**) secreted by splenocytes of progeny sensitised to birch pollen and stimulated with the mitogen concanavalin A (Con A) from mothers having received ST11 (black bars) or from control mice (white bars). *Ex vivo* proliferation of allergen-induced (Bet v 1) or ConA-induced (ConA) spleen cells **(C)** and MLN (**D**), isolated from the progeny of mothers treated (black bars) or not (white bars) with *L. paracasei* ST11.

### Detailed description

### Definitions:

In this specification, the following terms have the following meanings:
"*Weaning period*" is the period during which infants are adapting from pure liquid nutrition to solid or semi-solid foods, and adapting from quasi unique food type (generally mother milk or infant formula) to a variety of foods.
"*Sensitization*" means induction/development of allergen-specific IgE antibodies.
"*Non allergen-specific immune responses*" means cytokine production upon stimulation of immune cells by Concanavalin A (Con A), which is a mitogen stimulating lymphocytes i.e. committed T cells, in a non specific manner. Thus, the term "non allergen-specific immune response" is synonymous with "mitogen induced immune response".
"*Probiotic*" means microbial cell preparations or components of microbial cells with a beneficial effect on the health or well-being of the host. (Salminen S, Ouwehand A. Benno Y. et al "Probiotics: how should they be defined" Trends Food Sci. Technol. (1999):10 107-10). The definition of probiotic is generally admitted and in line with the WHO definition. The probiotic can comprise a unique strain of micro-organism, a mix of various strains and/or a mix of various bacterial species and genera. In case of mixtures, the singular term "probiotic" can still be used to designate the probiotic mixture or preparation. For the purpose of the present invention, micro-organisms of the genus *Lactobacillus* are considered as probiotics.
"*Prebiotic*" generally means a non digestible food ingredient that beneficially affects the host by selectively stimulating the growth and/or activity of micro-organisms present in the gut of the host, and thus attempt to improve host health.

*Lactobacillus paracasei* (*L. paracasei*) strain NCC 2461 (Nestlé Culture collection) is the *L. paracasei* with the alternative name of ST11, used throughout the text and the international identification reference CNCM I-2116 (Collection Nationale de Cultures de Microorganismes at Institute Pasteur, Paris, France). The CNCM identification refers to the Collection Nationale de Cultures de Microorganismes at Institut Pasteur, 22 rue du docteur Roux, 75724 Paris, France.

The present invention provides a probiotic, ST11, for use by administration to expectant mothers, and to their progeny for the reduction or prevention of the development of allergic immune responses in progeny. Thus, the invention provides a method for primary prevention or reduction of development of allergies in infants. All infants may benefit from the present invention, including those at risk of developing atopic diseases because of their family history.

According to the invention, a daily dose of ST11 is administered to a pregnant woman for at least two weeks before delivery and, after delivery, the daily dose of ST11 is administered to the newborn infant for at least two months, either directly or via the mother's milk.

Consequently, the invention also relates to a composition comprising ST11, for use by administration to expectant mothers, and to their progeny, according to the regimen as indicated above, for the reduction or prevention of the development of allergic immune responses in progeny.

By administering the ST11 according to the present invention, the intestinal microbiota of the infant is affected propitiously. By administering the ST11 to the mother during pregnancy and after delivery to infant, the immune responses to allergens are reduced, thus the allergic response is "dampened" and allergy, including atopy, may be prevented. This effect is elaborated upon in the paragraphs below.

### Doses of probiotic:

The daily doses of ST11 administered to the expectant or breast feeding mother is from 1x10⁶ to 1x10¹¹ cfu, preferably 1x10⁸ to 1x10¹⁰ cfu (cfu = colony forming unit). The daily dose suitable for newborn babies ranges from 1 x10² to 1 x10¹⁰, preferably, 1 x10² to 1x10⁴ cfu.

Thus ST11 may be present in the composition in a wide range of percentages provided that it delivers the immunity protective effect described. However, preferably, the ST11 is present in the composition in an amount equivalent to between 1 x10² and 1x10¹¹ cfu/g of dry composition. This includes the possibilities that the bacteria are live, inactivated or dead or even present as fragments such as DNA or cell wall materials. In other words, the quantity of bacteria which the formula contains is expressed in terms of the colony forming ability of that quantity of bacteria as if all the bacteria were live irrespective of whether they are, in fact, live, inactivated or dead, fragmented or a mixture of any or all of these states. Preferably, for administration to the expectant or lactating mother, the probiotic is present in an amount equivalent to between 1x10⁴ to 1x10⁹ cfu/g of dry composition, even more preferably in an amount equivalent to between 1x10⁶ to 1x10⁸ cfu/ g of dry composition.

The amount of probiotic present per gram of dry composition for administration to the progeny may be lower and the daily doses described above should be respected.

### Method of administration:

(i) Administration to expectant mothers:
   The composition can be administered to the expectant mothers by various ways as long as it induces a contact between the composition and the gastro-intestinal tract of the females. Preferably, the composition is orally administered as part of the food, drinks or dietary supplements of the expectant mothers. The composition can also be administered in a pharmaceutical composition. Preferably the administration is oral. However, in pathological conditions or when enteral feeding is otherwise used, the administration of the composition can be added to the enteral feeding.
(ii) Administration to newborn progeny:
   The ST11 can also be administered orally directly to the progeny alone (pure or diluted in water or mother's milk for example) as a food supplement or as an ingredient in an infant milk formula. Such a formula may be an infant "preterm formula" if the progeny is born before term or has a low birth weight, a "starter formula" or a "follow-on formula". The formula may also be an hypoallergenic (HA) formulas in which the cow milk proteins are hydrolysed. An example of such starter formula is given in **Example 2.**

### Administration with other compounds:

The ST11 can be administered alone (pure or diluted in water or milk, including breast milk for example) or in a mixture with other compounds (such as dietary supplements, nutritional supplements, medicines, carriers, flavours, digestible or non-digestible ingredients). Vitamins and minerals are examples of typical dietary supplements. In a preferred embodiment, the composition is administered together with other compounds that enhance the described effect on the immunity of the progeny. Such synergistic compounds may be carriers or a matrix that facilitates the ST11 delivery to the intestinal tract of the expectant mother or they may otherwise enhance the effect of the composition on the immune system of the progeny. Such compounds can be other active compounds that synergistically or separately influence the immune response of the infant and/or potentiates the effect of the probiotic. An example of such synergistic compounds is maltodextrin. One of the effect of maltodextrin is to provide a carrier for the probiotic, enhancing its effect, and to prevent aggregation.

Other examples include known prebiotic compounds such as carbohydrate compounds selected from the group consisting of inulin, fructooligosaccharide (FOS), short-chain fructooligosaccharide (short chain FOS), galacto-oligosaccharide (GOS), xylooligosaccharide (XOS), glangliosides, partially hydrolysed guar gum (PHGG) acacia gum, soybean-gum, apple extract, lactowolfberry, wolfberry extracts or mixture thereof. Other carbohydrates may be present such as a second carbohydrate acting in synergy with the first carbohydrate and that is selected from the group consisting of xylooligosaccharide (XOS), gum, acacia gum, starch, partially hydrolysed guar gum or mixture thereof. The carbohydrate or carbohydrates may be present at about 1 g to 20g or 1% to 80% or 20% to 60% in the daily doses of the composition. Alternatively, the carbohydrates are present at 10% to 80% of the dry composition.

The daily doses of carbohydrates, and all other compounds administered with the ST11 should always comply with the published safety guidelines and regulatory requirements. This is particularly important with respect to the administration to newborn babies.

In one embodiment, a nutritional composition preferably comprises a source of protein. Dietary protein is preferred as a source of protein. The dietary protein may be any suitable dietary protein, for example animal proteins (such as milk proteins, or meat proteins), vegetable proteins (such as soy proteins, wheat proteins, rice proteins or pea proteins), a mixture of free amino acids, or a combination thereof. Milk proteins such as casein and whey proteins are particularly preferred.

There is consensus in ongoing research leading to current recommendations to introduce potentially allergenic foods to babies at the weaning period i.e. in the first 3-7 months of life, while the baby is still at least partially breastfed. We strongly believe that the administration of ST11 to infants (either via breast feeding or by direct administration) facilitates the introduction of potentially allergenic foods, such as milk proteins, into the diet of the child.

The composition may also comprise a source of carbohydrates and/or a source of fat.

If the composition of the invention is a nutritional composition and includes a fat source, the fat source preferably provides about 5% to about 55% of the energy of the nutritional composition; for example about 20% to about 50% of the energy.

Lipid making up the fat source may be any suitable fat or fat mixture. Vegetable fat is particularly suitable, for example soy oil, palm oil, coconut oil, safflower oil, sunflower oil, corn oil, canola oil, lecithin and the like. Animal fat such as milk fat may also be added if desired.

An additional source of carbohydrate may be added to the nutritional composition. It preferably provides about 40% to about 80% of the energy of the nutritional composition. Any suitable carbohydrate may be used, for example sucrose, lactose, glucose, fructose, corn syrup solids, maltodextrin, or a mixture thereof. Additional dietary fibre may also be added if desired. If added, it preferably comprises up to about 5% of the energy of the nutritional composition. The dietary fibre may be from any suitable origin, including for example soy, pea, oat, pectin, guar gum, acacia gum, fructooligosaccharide or a mixture thereof. Suitable vitamins and minerals may be included in the nutritional composition in an amount to meet the appropriate guidelines.

One or more essential long chain fatty acids (LC-PUFAs) may be included in the composition. Examples of LC-PUFAs that may be added are docosahexaenoic acid (DHA) and arachidonic acid (AA). The LC-PUFAs may be added at concentrations so that they constitute greater than 0.01% of the fatty acids present in the composition.

One or more food grade emulsifiers may be included in the nutritional composition if desired; for example diacetyl tartaric acid esters of mono- and di- glycerides, lecithin and mono- or di-glycerides or a mixture thereof. Similarly suitable salts and/or stabilisers may be included. Flavours can be added to the composition.

### Administration period:

The expectant mother may start to take the ST11 as soon as she is aware of her pregnancy. However, the administration period may also start before pregnancy starts, for example if the female is trying to become pregnant. Administration may start at any time after the pregnancy starts. It may start relatively late in the pregnancy, preferably at month 3, 4, 5, 6, 7 or 8 of the pregnancy, in the case of human pregnancy, or in corresponding periods for other mammals, or up to two weeks before the expected delivery date.

The period of administration can be continuous (for example, up to and including lactation up to weaning), or discontinuous. Continuous administration is preferred for a more sustained effect. However, it is speculated that a discontinuous pattern (for example, daily administration during one week per month, or during alternate weeks) can induce positive effects on the progeny.

The duration of the administration may vary. While positive effects are expected with relatively short duration of administration (for example, daily administration during one week for newborns and one or two months for adults), longer durations are believed to provide enhanced effect (for example, a duration of three, five or eight months in humans, and corresponding periods in other mammals).

The administration should cover at least part of the gestation period and at least part of the lactation period, or the equivalent period should the newborn not be breastfed. Preferably, the administration period to the expectant mother covers substantially the full length of the gestation period, although this may be less. Similarly, the administration period for the lactating mother preferably covers substantially the full length of the lactation period, although, again, this period may be less.

Preferably, the administration to the mother is by daily intake (to be taken once or twice a day), or weekly intake (to be taken one or twice a week).

The ST11 may be administered to the infant directly. This is the case particularly if the mother does not breastfeed or after she discontinues breastfeeding. However, an infant who is being breastfed may also receive the ST11 by direct administration. The administration to the infant, either via breastfeeding, or by direct administration, or both methods, may be continued up until the age of six months or even one year. Thus, the ST11 may be administered during lactation if lactation takes place, or after partial or full weaning.

Preferably, the administration to the infant is by daily intake (to be taken once or twice a day), or weekly intake (to be taken one or twice a week).

### Effect of the probiotic administration:

ST11 administered to expectant mothers protects the progeny from the development of allergy. Thus the progeny of ST11 treated mothers who were sensitised with a specific allergen and then challenged with the same allergen had a reduction in allergic response (in this case, an allergen specific recall response) compared to progeny of non-treated mothers. Similarly, when stimulated with a mitogen, a reduced level of immune response (i.e. a non-specific response) was observed in the progeny of the treated mothers.

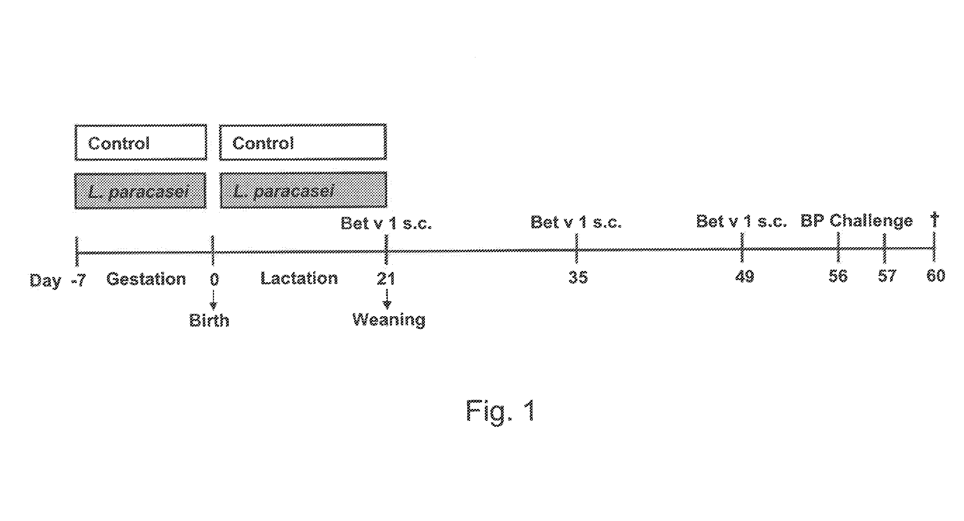

In one embodiment of the invention, maternal ST11 administration reduces the development of airway inflammation in progeny which have been sensitised to air borne allergens, for example birch pollen (see **Example 1,** **Figure 1** for study outline) and then challenged to the same allergen. This may be demonstrated by lower eosinophilic infiltration into the airway lumen in comparison to sensitized and challenged progeny from mothers who have not been treated with ST11 (see **Fig. 3A**).

The inventors have also demonstrated that maternal ST11-supplementation does not have an adverse effect on the progeny's ability to fight infection. While the number of eosinophils- these are cells directly implicated in mounting an allergic response- are reduced, the number of macrophages and lymphocytes in bronchoalveolar lavage (BAL) fluid of progeny of **Example 1** are not affected (see **Fig. 3A**) compared to progeny of ST11 non treated mothers.

Histological analysis of lung sections revealed that airway inflammation, goblet cell metaplasia and mucus production were reduced in lungs of the progeny of ST11 - treated mothers in contrast to progeny of control mothers (See **Figure 2**). Progeny of control mice showed massive cellular infiltration around bronchi and blood vessels (Fig. **2B, E**). Considerably reduced peribronchial inflammation with only rare mononuclear cell infiltrates were detected in progeny derived from ST11-exposed mothers (Fig. **2C, F**). No infiltrates were found in the lungs of naïve animals (Fig. **2A, D**). In accordance, periodic acid-Schiff (PAS) staining of lung sections revealed that the development of goblet cell hyperplasia and mucus hypersecretion was strongly reduced in progeny of ST11-treated mothers (**Fig. 2F**) as compared to the control group, whose mothers received water instead of ST11, (Fig. **2E**). Lung sections from naïve mice showed no mucus secretion (Fig. **2A, D**). Taken together, these data suggest that maternal exposure to ST11 results in changes in the mucosal immune responses in progeny such that subsequent stimulation with allergen results in remote airway inflammation.

In another embodiment of the invention, maternal ST11 administration reduces the levels of interleukin 5 (IL-5) in BAL fluid (**Fig. 3B**), lung (**Fig. 3C**) and lung associated lymph nodes (**Fig. 3D**) in mice which have been sensitised to an air borne allergen, for example Bet v 1 (**Example 1**) and then aerosol challenged with birch pollen. The levels of IL-5 were found to be significantly higher in the progeny of mothers which were not treated with ST11. Thus, maternal ST11 administration reduces allergen specific recall response in progeny.

To test whether maternal ST11 supplementation might have a long lasting immune imprinting in the progeny by changing cellular Th1/Th2 balance, splenocytes of sensitized and challenged progeny were stimulated with Bet v 1. Considerably lower levels of Th2 cytokines (IL-4 and IL-5) secretion were observed in splenocytes from the progeny of ST11-supplemented mothers compared with progeny derived from control mothers (**Fig. 4A****, B**). Moreover, stimulation of spleen cells from perinatal sham-treated (control group) progeny with Bet v 1 in the presence of ST11 *in vitro* led to significant reduction of Bet v 1-induced IL-4 and IL-5 (**Fig. 4C****, D**).

The suppressive effect of ST11 on IL-5 production, which is key for the recruitment and survival of eosinophils, provides a mechanistic explanation for the reduced airway eosinophilia in progeny of ST11 -treated mothers.

Thus maternal ST11 administration reduces allergen specific recall response.

In another embodiment of the invention, maternal ST11 administration reduces the levels of interleukin 5 (IL-5) secretion in splenocytes of progeny sensitised to birch pollen and stimulateded with the mitogen Concanavalin A (Con A)(**Example 1**). Con A is a lectin known for its ability to stimulate T cell subsets giving rise to functionally distinct T cells populations (Dwyer et al. (1981), Clin Exp Immunol 46(2): 237-249). IL -4 levels remained unchanged (see **Figure 5A****, B**).

Thus, maternal ST11 supplementation reduces mitogen-induced as well as allergen specific cellular immune responses.

The inventors have found that perinatal ST11-supplementation led to reduced allergen-induced and mitogen-induced proliferative responses of spleen and mesenteric lymph node (MLN) cells *ex vivo* (**Fig. 5E and F,** respectively). Thus, this suggests that maternal application of ST11 may lead to an integrated imprinting of the immune system of the neonate mice, modulating cellular responses following antigen-specific and non-specific stimulation.

According to the invention, the reduction or prevention of the development of allergic immune responses in the progeny of ST11-treated mothers may be the reduction or prevention of allergic immune responses to air borne allergens, food allergens or contact allergens such as nickel. The air borne allergens may be house dust mites, animal danders, and tree and grass pollen, especially birch pollen. Bet v 1, Bet v 2 profilins and cross reactive carbohydrate determinants (CDD) are some of the known allergens of birch pollen.

According to the invention, the reduction or prevention of the development of allergic immune responses in progeny of ST11-treated mothers may be the reduction or prevention of allergic immune responses to allergens that are cross reactive with birch pollen. These may be food allergens such as apples, stone fruits, celery, carrot, nuts, and soybeans that may contain allergens that are cross-reactive to Bet v 1. These also may be peach, orange, lychee fruit, strawberry, persimmon, zucchini, and carrot as these are known to be reactive to the Bet v 2 allergen.

According to the invention, the reduction or prevention of the development of allergic immune responses in progeny of ST11-treated mothers may be a reduction of at least 5 %, more preferably 10%, more preferably at least 30% and most preferably at least 50% compared to the progeny of non treated mothers.

The ST11 strain is a probiotic isolated from the fecal microbiota of a breastfed child. Thus, with respect to a health promoting strategy trying to mimic as closely as possible the microbiota of breastfed children, the administration of ST11, particularly to non-breast fed children, may provide an advantage over other strains that are not found in breastfed children.

### Example 1:

A mouse model of birch pollen allergy was used to demonstrate that maternal supplementation with ST11 protects progeny from the development of an allergic phenotype.

BALB/c mice received ST11 daily in drinking water during the last week of gestation and for the period of lactation. The pregnant and lactating female mice mothers were fed daily with the live probiotic bacterial strain ST11 in drinking water. Progeny of ST11 - or sham-treated mothers was sensitized with the recombinant major birch pollen allergen Bet v 1 and challenged with birch pollen extract (**Fig. 1** Experimental design).

### Animals:

Pregnant BALB/c mice (day 14 of pregnancy) were purchased from Charles River (Sulzfeld, Germany) and maintained under conventional housing conditions. Female TLR2- and TLR4-deficient and wild type mice with C57BL/6 genetic background. were obtained from the University of Veterinary Medicine of Vienna (Austria).All experiments were approved by the Animal Experimentation Committee of the University of Vienna and by the Federal ministry of Science and Research.

### Probiotic bacteria:

Probiotic strain ST11 is *L. paracasei* NCC 2461 CNCM I-2116 (*L. paracasei*) and belongs to Nestlé Research Center bacterial collection (Lausanne, Switzerland). For perinatal oral application, spray-dried bacterial preparation diluted in drinking water was used. For *in vitro* experiments, *L. paracasei* preparation was inactivated with 1% formaldehyde-PBS for 3 h at room temperature, washed twice with sterile PBS, and stored at -20°C.

### Perinatal ST11 exposure:

Pregnant BALB/c mice received 5x10⁸ cfu/ml of ST11 in drinking water every day (reaching average daily dose of 2x10⁹ cfu/mouse) for a total of 4 weeks, starting from the third trimester of gestation (day -7) and continuing throughout the lactation period until the day of the weaning (day 21) (Experimental design; **Fig. 1**). Age-matched control animals received only water.

The presence of ST11 in the faeces was tested by PCR analysis. DNA was prepared from faecal samples and amplified using ST11 specific PCR primers. As expected, ST11-specific PCR products were detected exclusively in the samples from ST11-treated, but not from control mother mice (data not shown). Stool samples from progeny were collected on day 21 of age (weaning). PCR analysis revealed that ST11 was not present in progeny of ST11 -treated or control mothers (data not shown).

### Sensitization and challenge of progeny:

Progeny from perinatally ST11-exposed or control-treated mother mice were sensitized three times subcutaneously (s.c.) with 1µg of recombinant major birch pollen allergen Bet v 1 (rBet v 1; Biomay, Vienna, Austria) emulsified in aluminium hydroxide (alum, Serva, Heidelberg, Germany) in a total volume of 150µl on days 21, 35 and 49 (Experimental design; **Fig. 1**). To induce airway inflammation, mice were challenged 1 week after the last sensitization by exposure to aerosolized 1% birch pollen extract (BP; Allergon, Välinge, Sweden) on two consecutive days (days 56-57). Mice were terminally anesthetized with Sevofluran (Abbott, Vienna, Austria) 72 h after final airway challenge (day 60).

### BAL and differential cell counts:

Progeny from ST11- or control-treated mother mice were terminally anesthetized and BAL was collected two times using 2 ml PBS containing protease inhibitor cocktail (Roche, Mannheim, Germany). Total leukocytes were counted and cytospins (Shadon Cytospin^{®}, Shadon Southern Instruments, USA) were stained with haematoxylin and eosin (H&E; Hemacolor^{®}, Merck, Darmstadt, Germany).

Standard morphological criteria were used to identify cell types and 200 cells were counted per cytospin via light microscopy. Cell-free supernatants were stored at - 20°C for further analysis.

### Lung histology :

Whole lungs were removed from mice and fixed with a 10% formaldehyde-PBS for histology. Paraffin-embedded tissue sections were stained with H&E. Airway mucus occlusions were analyzed on periodic acid-Schiff-stained (PAS, Sigma-Aldrich) sections.

### Ex vivo stimulation of cell cultures:

Spleens, lungs, lung draining mediastinal lymph nodes (Lung LN), and mesenteric lymph nodes (MLN) were collected on sacrifice and single-cell suspensions were prepared from all organs. Where indicated, cells (2.5 x 10⁷/ml) were stimulated with rBet v 1 (10µg/ml) or Concanavalin A (ConA) (1µg/ml, Sigma-Aldrich) in 96-well plates at 37°C for 72 hours in culture medium (RPMI 1640 supplemented with 10% heat-inactivated FCS, 2 mM L-glutamine 100 U/ml penicillin, 100 µg/ml streptomycin). As control, cells were cultured with media alone. Proliferative responses of spleen and MLN cell cultures were determined by scintillation counting after addition of ³[H]-thymidine (0.5µCi/well, Amersham, Buchter, Braunschweig, Germany) for the last 18 h. Moreover, to investigate the immunomodulatory potential of ST11 *in vitro,* spleen cells of progeny sham-treated mothers were incubated with ST11 (10⁷ cfu/ml) admixed to rBet v 1 (10µg/ml) for 48 h.

### Cytokine measurements:

Levels of IL-4 and IL-5 were measured with ELISA kits (Endogen, Cambridge, MA) according to the manufacturer's instructions.

### Statistical analysis:

All data are shown as means ± SEM. Significance was analyzed by using Graph Prism software (Graph Pad Softwar, Inc, San Diego, CA) and the non-parametric Mann-Whitney U-test. Significant differences were considered at p<0.05.

### Example 2:

An example of the composition of an infant formula for use according to the present invention is given below. This composition is given by way of illustration only. The protein source is a conventional mix of whey protein and casein.

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Prebiotic (100% GOS) (g) | 0.64 | 4.3 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| Lactobacilus paracasei NCC 2461 (ST11; see experimental part) | 2x10⁷ cfu/g of powder | |

## Claims

1. *Lactobacillus paracasei* NCC 2461 (ST11) for use by administration to expectant mothers and, optionally, to same mothers while lactating, and to progeny born of said mothers in reduction or prevention of the development of allergic immune responses in progeny.

2. *Lactobacillus paracasei* NCC 2461 (ST11) of claim 1 wherein the progeny are at risk of atopic diseases.

3. *Lactobacillus paracasei* NCC 2461 (ST11) of claims 1 or 2 wherein the allergic immune responses are to food and/or airborne allergens.

4. *Lactobacillus paracasei* NCC 2461 (ST11) of claim 3 wherein said allergic immune responses are to airborne allergens, especially birch pollen allergens Bet v 1 and Bet v 2.

5. *Lactobacillus paracasei* NCC 2461 (ST11) of claim 3 wherein the allergic immune responses are to food allergens that are cross-reactive with tree allergens, preferably birch pollen allergens Bet v 1 and Bet v 2.

6. *Lactobacillus paracasei* NCC 2461 (ST11) of claim 5 wherein the food allergens are apple, pear, stone fruits, celery, carrot, nuts and soybeans.

7. *Lactobacillus paracasei* NCC 2461 (ST11) of any of the preceding claims wherein said allergic immune responses may be allergen-specific recall responses or non allergen-specific immune responses.

8. *Lactobacillus paracasei* NCC 2461 (ST11) of the preceding claims wherein the reduction of said allergic immune responses in said progeny is
(i) a reduction in IL-4 and IL-5 production, in the case of allergen specific recall responses and/or
(ii) a reduction in IL-5 production in the case of non allergen-specific immune responses
the reduction in responses being with respect to progeny of mothers not treated with ST11.

9. *Lactobacillus paracasei* NCC 2461 (ST11) according to any of claims 1-8 wherein the ST11 is administered to expectant mothers for at least two weeks before delivery and, after delivery, to the progeny either directly or via mothers milk, for at least 2 months, preferably up to six months after delivery.

10. *Lactobacillus paracasei* NCC 2461 (ST11) according to claim 9 wherein the ST11 is administered orally to the expectant or lactating mothers, preferably in foods, drinks, dietary supplements or pharmaceutical compositions.

11. *Lactobacillus paracasei* NCC 2461 (ST11) according to claim 10 wherein the ST11 is administered to the progeny via the mother's milk or directly to the infant as a supplement, in its pure form or diluted in water, breast milk or in an infant formula.

12. *Lactobacillus paracasei* NCC 2461 (ST11) of any of the preceding claims wherein said ST11 is administered to the expectant or lactating mother in a daily dose of from 1 x10⁶ to 1x10¹¹ cfu, preferably 1x10⁸ to 1 x10¹⁰ cfu (cfu = colony forming unit).

13. *Lactobacillus paracasei* NCC 2461 (ST11) of any of claims 1-11 wherein said ST11 is administered to the progeny as a daily dose of from 1x10² to1x10¹⁰, preferably 1x10² to1x10⁴ cfu (cfu = colony forming unit).

14. *Lactobacillus paracasei* NCC 2461 (ST11) of any of the preceding claims wherein said ST11 is administered to the mother or baby as a composition comprising between 10² and 10⁸ cfu per gram of dry composition.

15. *Lactobacillus paracasei* NCC 2461 (ST11) of claim 14, wherein said composition comprises further ingredients or prebiotics, preferably selected from inulin, fructooligosaccharide (FOS), short-chain fructooligosaccharide (short chain IOS), galacto-oligosaccharide (GOS), xylooligosaccharide (XOS), glanglioside, partially hydrolysed guar gum, acacia gum, soybean-gum, Lactowolfberry, wolfberry, apple extracts or mixture thereof.

16. *Lactobacillus paracasei* NCC 2461 (ST11) of any of the preceding claims wherein said ST11 has been inactivated such as to render it non replicating.

17. Method for primary prevention or reduction of the development of allergic immune responses in infants, comprising
- administering to a pregnant woman a daily dose of live *Lactobacillus paracasei* NCC 2461 (ST11) for at least two weeks before delivery, and
- after delivery, administering to the newborn infant a daily dose of live probiotic bacteria for at least 2 months, preferably up to six months or one year after delivery.

18. The method according to claim 17, wherein, after delivery, *Lactobacillus paracasei* NCC 2461 (ST11) is administered via the breastfeeding mother.

19. The method according to claim 17 or 18, wherein the daily dose to the expectant or lactating mother is approximately from 1x10⁶ to 1x10¹¹ cfu, preferably 1x10⁸ to 1x10¹⁰ cfu (cfu = colony forming units) of ST11.

20. The method according to claim 17 or 18 wherein the daily dose to progeny is approximately 1x10²-1x10¹⁰, preferably 1x10² to 1x10⁴ cfu (cfu = colony forming units) of *Lactobacillus paracasei* NCC 2461 (ST11).
